# EUROPÄISCHE PATENTSCHRIFT

(11) **EP 4 008 233 B1**
(45) Veröffentlichungstag und Bekanntmachung des Hinweises auf die Patenterteilung: **15.01.2025**
(21) Anmeldenummer: 21205458.9
(22) Anmeldetag: 29.10.2021
(51) Int. Cl.: A61B 1/00, A61B 1/04

(54) **ENDOSKOPISCHE VORRICHTUNG, VERFAHREN ZUR ÜBERPRÜFUNG EINER IDENTITÄT EINER KOMPONENTE EINER ENDOSKOPISCHEN VORRICHTUNG UND COMPUTERPROGRAMMPRODUKT**
ENDOSCOPIC DEVICE, METHOD FOR VERIFYING THE IDENTITY OF A COMPONENT OF AN ENDOSCOPIC DEVICE AND COMPUTER PROGRAM PRODUCT
DISPOSITIF ENDOSCOPIQUE, PROCÉDÉ DE VÉRIFICATION D'UNE IDENTITÉ D'UN COMPOSANT D'UN DISPOSITIF ENDOSCOPIQUE ET PRODUIT-PROGRAMME INFORMATIQUE

(30) Priorität: 07.12.2020 DE 102020132454
(43) Veröffentlichungstag der Anmeldung: 08.06.2022
(73) Patentinhaber: Karl Storz SE & Co. KG, 78532 Tuttlingen (DE)
(72) Erfinder: Hinding, Thomas, 78532 Tuttlingen (DE)
(74) Vertreter: Weidner Stern Jeschke

(56) Entgegenhaltungen:
- DE-A1- 102016 214 990
- DE-U1- 20 213 926
- US-A1- 2005 159 646
- US-B1- 6 537 207

## Beschreibung

Die Erfindung betrifft eine endoskopische Vorrichtung, insbesondere medizinische oder industrielle endoskopische Vorrichtung, wobei die endoskopische Vorrichtung als Komponenten eine Lichtquelle, einen Lichtleiter, eine Kamera und ein Endoskop mit einem optischen Element oder mehreren optischen Elementen aufweist. Des Weiteren betrifft die Erfindung ein Verfahren zur Überprüfung einer Identität einer Komponente einer endoskopischen Vorrichtung und ein Computerprogrammprodukt zum Auswerten einer Bildaufnahme einer optischen Markierung eines optischen Elementes und zum Identifizieren eines Endoskops einer endoskopischen Vorrichtung.

In medizinischen und nicht-medizinischen Anwendungen werden Endoskope mit einem langen Schaft verwendet, um diesen in eine innere Kavität eines menschlichen oder tierischen Körpers oder in ein anderes Objekt für eine Untersuchung einzuführen, wie beispielsweise in eine Rohrleitung. Neben dem eigentlichen Endoskop umfasst eine endoskopische Vorrichtung für solche Einsätze weitere Komponenten, wie eine Lichtquelle, einen Lichtleiter zum Verbinden der Lichtquelle mit dem Endoskop und eine Kamera. Häufig passen die Komponenten aus verschiedenen Baureihen und/oder für verschiedene Anwendungen eines Herstellers oder sogar mehrerer Hersteller untereinander mechanisch zusammen. Zudem werden im Handel Adapter frei angeboten, welche eine mechanische Kopplung der Komponenten von endoskopischen Vorrichtungen vom selben Hersteller oder unterschiedlicher Hersteller ermöglichen.

Dadurch kann es leicht zu einer Verwendung der endoskopischen Vorrichtung mit einer nicht optimal zusammengestellten und/oder nicht für einen bestimmten Einsatz vorgesehenen Kombination ihrer Komponenten kommen. Dies kann zu ernsthaften Problemen während der Anwendung führen; wenn beispielsweise ein zu großer Lichtleiter in einem zu kleinen Endoskop verwendet wird, entsteht zu viel Hitze an der Koppelstelle. Auch funktionieren einige Kombinationen prinzipiell nur mangelhaft, zum Beispiel ein Laparoskop mit einem Lichtleiter mit 2 mm Durchmesser. Eine ungeeignet zusammengesetzte Vorrichtung kann erhebliche Konsequenzen für einen Patienten haben und zum Stellen einer falschen Diagnose führen, wenn beispielsweise ein Fluoreszenzfarbstoff im Rahmen der photodynamischen Diagnostik (PDD) verwendet wird oder die Nahinfrarotfluoreszenz unter Verwendung von Indocyaningrün (ICG) eingesetzt wird und aufgrund nicht optimal Kombination der Komponenten die entsprechenden Farbsignale nicht optimal empfangen, dargestellt und/oder erkannt werden können.

Prinzipiell könnte bei einem Design von neuen Vorrichtungen mit einem Endoskop und einer Kamera ein Inkompatibilitätsschutz eingebaut werden, um eine Verbindung mit früheren und/oder anderen Geräten und Instrumenten zu unterbinden. Dies ist jedoch weder für den Anwender, noch für den Hersteller der endoskopischen Geräte wirtschaftlich und praktisch durchführbar. Zudem kann es vorkommen, dass unter einem endoskopischen Eingriff die Art des zunächst verwendeten Endoskops gewechselt werden muss, weil beispielsweise der Eingriff auf andere Organe erweitert werden muss. Folglich muss auch aus praktischen Gesichtspunkten eine Austauschbarkeit der Komponenten einer endoskopischen Vorrichtung gegeben sein, solange diese in einer Kombination resultiert, welche für den jeweiligen Einsatz geeignet ist.

Zudem werden in klinischen Abläufen eine Vielzahl von medizinischen Instrumenten gehandhabt und verwendet. So müssen in der Vor- und Nachbereitungsphase einer chirurgischen Operation die benötigten Instrumente bereitgestellt, überprüft und anschließend einer Entsorgung oder Aufbereitung (Sterilisation) zugeführt werden. Dazu werden die medizinischen Instrumente üblicherweise mit einer äußeren Markierung versehen, wie beispielsweise einem eindimensionalen Code (Strichcode, Barcode) oder zweidimensionalen Code (Matrixcode), welche mit einem zugehörigen optischen Lesegerät eingelesen werden können. Des Weiteren sind RFID-Transponder (Radio Frequency Identification) oder RFID-Tags zur Identifizierung von chirurgischen Instrumenten bekannt, wobei auch hierbei die Identifizierung mit einem korrespondierenden externen Lesegerät ausgelesen werden muss.

Aus der DE 10 2013 002 832 A1 ist ein Roboter mit mindestens einem beweglichen Armelement bekannt, bei dem zur automatischen Instrumentenerkennung des Typs des am Roboter befestigten Instrumentes das chirurgische Instrument einen RFID-Chip oder einen Barcode zur optischen Erkennung aufweist.

In der DE 10 2014 109 888 A1 ist ein Verfahren und eine Vorrichtung zum Prüfen der Licht- und/oder Bildübertragungseigenschaften eines endoskopischen oder exoskopischen Systems beschrieben, wobei die Identifikationsdaten eines Identifikators des Endoskops oder Exoskops und/oder des Lichtträgers berührungslos mittels RFID-Technologie oder optisch, beispielsweise über Barcode und Barcodeleser, extern ausgelesen werden.

In der DE 10 2015 016 233 A1 wird ein ringfömiger RFID-Transponder zur Markierung eines Endoskopes vorgeschlagen, welcher beispielwiese in einer Okularmuschel des Endoskopes angeordnet ist.

Die EP 2 390 645 B1 beschreibt eine Vorrichtung zum Testen eines Endoskops, wobei das Endoskop beweglich relativ zu und vor einem Testbild auf einem Testbildhalter angeordnet ist, wobei das Endoskop Identifikationselemente, wie einen Barcode, und die Vorrichtung zum Testen korrespondierende Leseelemente, wie einen Barcodescanner, zum Lesen der Identifikationselemente aufweist.

Die DE 10 2013 101 158 A1 betrifft eine medizinische Vorrichtung zur Bildung eines medizinischen Systems aus einer oder mehreren medizinischen Einrichtungen und entsprechenden Kupplungsstellen, bei der ein- oder zweidimensionale Barcodes an einem Ende einer Datenleitung, eines Lichtleiterkabels, einer elektrischen Leitung oder eines Ablassschlauches mittels eines Barcode-Scanners, einer Kamera mit Bilddatenverarbeitungseinrichtung oder einer RFID-Leseeinrichtung erfasst werden.

In der EP 2 641 552 A2 umfasst ein chirurgisches Instrument einen Instrumentenkörper, welcher betriebsbereit mit verschiedenen Arten von zu kommunizierenden Endeffektor-Baugruppen koppelbar ist, und eine modulare Endeffektoranordnung ein abnehmbares Steuermodul aufweist, welches die Stromversorgung an die Endeffektor-Baugruppe basierend auf der jeweiligen Art der Endeffektoranordnung steuert. Für die Kopplung können verschiedene Schäfte und/oder Endeffektoren einen jeweiligen Farbcode umfassen, um zwischen verschiedenen Längen und/oder Typen zu unterscheiden.

Nachteilig bei außen an Komponenten einer mehrteiligen medizinischen oder industriellen Vorrichtungen angebrachten Identifikationscodes ist, dass diese entweder vom Benutzer fehleranfällig manuell überprüft werden müssen oder zusätzliche optische, elektronische und/oder kabellose Geräte zum Einlesen und/oder Erkennen des äußeren Codes notwendig sind. Zudem besteht bei außen angebrachten Codes die Gefahr, dass diese durch die mehrfache Aufbereitung und/oder Sterilisation der Komponente mit der Zeit verblassen und/oder nicht mehr lesbar sind.

In US 2013/0046299 A1 ist eine intelligente Elektrodenvorrichtung zur Überwachung der Verwendung einer elektrochirurgischen Elektrode beschrieben, wobei die elektrochirurgische Elektrode und ein Trackingelement innerhalb einer Verkleidung des intelligenten Elektrodensystems angeordnet sind. Das Trackingelement weist einen Mikrochip zur Speicherung von Prozessdaten der Elektrode auf. Nachteilig ist, dass ein Identifizieren der Elektrode nur während der Anwendung über einen Abgleich ihrer verarbeiteten Prozessdaten zu denen einer Seriennummer bekannter Elektroden erfolgt.

Des Weiteren sind optische Bilderfassungs- und Erkennungssysteme, beispielsweise mittels einer Kamera, bekannt.

In der DE 10 2014 217 095 A1 weist eine verbindbare Elektrode am distalen Ende eines chirurgischen Instrumentes ein optisch erfassbares äußeres Identifikationsmerkmal auf, wobei mittels eines Bilderfassungssystems zum Erfassen eines Operationsfeldes ein Bild der äußeren Elektrode erfasst wird und die Bilddaten an eine Datenverarbeitungseinheit übertragen werden, mit welcher in mittels einer Datenbank eine Zuordnung des Identifikationsmerkmales der Elektrode zu einem Elektrodentyp und einem vorgesehenen Betriebsparameter erfolgt.

Aus der DE 10 2006 054 148 B4 ist eine Vorrichtung zum optischen Erfassen von Störungen und/oder Codes in einem Untersuchungsbereich auf einer ebenen optischen Grenzfläche eines transparenten Körpers mit einer Beleuchtungseinrichtung, einer Bildaufnahmeeinrichtung und einer Bildverarbeitungseinrichtung zur Auswertung offenbart, wobei, im Falle von Codes, die aufgenommenen Bilder von der Bildverarbeitungsrechner mittels eines Zeichenerkennungsprogramms automatisch analysiert und decodiert werden. Als Bildaufnahmeeinrichtung wird beispielsweise eine extern außerhalb der optischen Achse der Vorrichtung angeordnete CCD-Kamera verwendet.

Die US 2008/0262654 A1 offenbart ein Robotersystem aufweisend einen Roboter und eine Aktuatoreinheit mit einer verbind- und lösbaren Arbeitseinheit, wobei die Arbeitseinheit im Verbindungsbereich einen zweidimensionalen Bildcode mit Informationen über die Arbeitseinheit aufweist und eine Kamera zur Erkennung des zweidimensionalen Bildcodes oder zur Bildaufnahme und LEDs zur Beleuchtung an der gegenüberliegenden Kontaktfläche der Aktuatoreinheit angeordnet sind, sodass mittels der Kamera ein Bild des zweidimensionalen Bildcodes aufgenommen werden kann.

In der DE 10 2017 103 804 A1 wird eine Abbildungsoptik einer Endoskopie-Kamera verwendet, um eine Information einzulesen, die zur Identifikation eines endoskopisch zu untersuchendem Objekt, beispielsweise eines Patienten, gezielt ausgerichtet wird. Hierzu wird die Abbildungsoptik der Endoskopie-Kamera beispielsweise auf einen einzulesenden Code auf einer Patientenkarte ausgerichtet. Mittels einer entsprechenden Programmierung einer zugehörigen Bildverarbeitungseinheit wird anschließend aus dem Bild des Codes eine Identifikationsinformation extrahiert.

Die US 6,537,207 B1 offenbart eine wegwerfbare Schutzabdeckung mit einem transparenten Kopf zum Verhindern von Kreuzinfektionen, welche außen auf ein distales Ende einer Sonde einer medizinischen Bildgebungsvorrichtung aufgesetzt und nach einer Verwendung im Patienten ausgetauscht wird. Die Schutzabdeckung weist visuelle Elemente oder Zeichen innerhalb eines Sichtfeldes des optischen Systems der Bildgebungsvorrichtung aufweist, um sicherzustellen, dass die richtige Schutzabdeckung in der exakten Position aufgesetzt ist.

US 2005/159646 A1) beschreibt eine Zubehörvorrichtung für eine optische Sonde, wobei die Zubehörvorrichtung mit einem Endoskop verbindbar ist und ein Strichcode auf einem optischen Fenster der Zubehörvorrichtung angebracht ist, welcher mit einem elektrischen Leser auslesbar ist.

Die DE 202 13 926 U1 offenbart eine Endoskop-Optik, bei welcher in einer proximalen Zwischenbildebene zwischen Stablinsen in einem Schaft und einer Bildbetrachtungseinrichtung eine Maske mit einem lichtdurchlässigen inneren Bereich zum Begrenzen des Bildes und einem lichtundurchlässigen äußeren Bereich angeordnet ist. In dem lichtundurchlässigen äußeren Bereich sind lichtdurchlässige Felder in Form von Strichcodierungen und Mustern ausgebildet, um das Instrument bestimmten Aufnahmen zu zuordnen.

In der DE 10 2016 214 900 A1 wird ein optisches System mit mehreren optischen Komponenten beschrieben, welche in einem Bereich außerhalb eines Strahlenganges jeweils mindestens eine Markierung mit einer Information zur einer räumlichen Anordnung der jeweiligen optischen Komponenten umfassen, um die optischen Achsen der optischen Komponenten zu berechnen und die optischen Komponenten zueinander zu zentrieren. Das Zentrieren der optischen Komponenten erfolgt in einer Einrichtung zum Montieren des optischen Systems unter Verwendung einer externen Kamera.

Nachteilig bei allen bekannten Vorrichtungen und Verfahren zur Identifikation einer medizinischen oder industriellen Vorrichtung ist vor allem, dass das äußere Anbringen eines Identifizierungscodes nicht manipulationssicher ist oder einem Verschleiß bei der Benutzung unterliegt. Zudem sind separate Geräte zum Auslesen des Identifizierungscodes oder separat angeordnete und/oder auszurichtende Kameras zum Erfassen eines entsprechenden Bildes des Identifizierungscodes sowohl bei außen als auch bei innen angeordneten Identifizierungscodes der bekannten Vorrichtungen notwendig.

Aufgabe der Erfindung ist es, den Stand der Technik zu verbessern.

Gelöst wird die Aufgabe durch eine endoskopische Vorrichtung, insbesondere medizinische oder industrielle endoskopische Vorrichtung, gemäß Anspruch 1, wobei die endoskopische Vorrichtung als Komponenten eine Lichtquelle, einen Lichtleiter, eine Kamera und ein Endoskop mit einem optischen Element oder mehreren optischen Elementen aufweist, und mindestens ein optisches Element eine optische Markierung zur Überprüfung einer Identität des optischen Elementes und/oder des Endoskops derart aufweist, dass die optische Markierung mit der Kamera zur Überprüfung der Identität und/oder einer Konfiguration der endoskopischen Vorrichtung erfassbar ist, wobei die Kamera ein Zoomobjektiv mit einem Verstellmittel zum Verändern einer Brennweite derart aufweist, dass mit dem Zoomobjektiv die optische Markierung oder die optischen Markierungen in einem Bereich von einem proximalen Ende bis zu einem distalen Ende des Endoskops jeweils in einer definierten Markierungszoomstellung erfassbar ist oder sind.

Dadurch wird eine endoskopische Vorrichtung bereitgestellt, welche an sich bei einer richtigen Konfiguration der Komponenten direkt für eine bestimmte medizinische oder industrielle Untersuchung einsetzbar ist, welche vorteilhaft selbst vor diesem Einsatz die Identität des optischen Elementes und/oder des Endoskopes überprüft. Somit wird eine automatische Komponenten- und/oder Instrumentenerkennung zur Überprüfung und/oder Überwachung der Identität einer Komponente oder mehrerer Komponenten, insbesondere des Endoskops, bereitgestellt, welches für einen bestimmten medizinischen oder industriellen Eingriff konfiguriert wurde.

Durch Erfassen der optischen Markierung als Identifikationscode werden die für einen bestimmten Einsatz erforderlichen und funktional miteinander verbundenen Komponenten der endoskopischen Vorrichtung erkannt und verifiziert, sodass ein medizinischer Eingriff oder eine industrielle Untersuchung ordnungsgemäß und fehlerfrei durchführbar ist. Folglich können ernsthafte Probleme, wie beispielsweise eine zu große Hitzeentwicklung an einer Koppelstelle von zwei nicht zueinander passenden Komponenten oder eine falsche Bildaufnahme bei einem bestimmten Licht- und/oder Fluoreszenzfarbstoff-Modus und folglich eine falsche Diagnose bei einem Patienten vermieden werden.

Gerade in Fällen, bei denen die verschiedenen Komponenten aus verschiedenen Baureihen eines Herstellers oder verschiedener Hersteller direkt oder mittels kommerziell verfügbaren Adaptern miteinander mechanisch koppelbar sind, ist durch das Erfassen der optischen Markierung einer Komponente die funktional richtige und/oder für einen bestimmten Einsatz optimale Verbindung und Konfiguration der endoskopischen Vorrichtung bestimmbar und/oder verifizierbar.

Es ist besonders vorteilhaft, dass die optische Markierung ein inhärentes und/oder beständiger Bestandteil der Komponente und/oder des Endoskopes ist, sodass dieses nicht einer Manipulation und/oder Fehleranfälligkeit unterliegt.

Hierzu ist die optische Markierung bevorzugt an und/oder in einem festen Bestandteil der Komponente angeordnet, wie beispielsweise einem optischen Element des Endoskops.

Vor allem ist es vorteilhaft, dass die bildgebende Kamera der endoskopischen Vorrichtung, welche direkt zur Bildaufnahme des zu untersuchenden Objektfeldes verwendet wird, ebenfalls zuvor direkt die optische Markierung des optischen Elementes erfasst. Dadurch ist eine zusätzliche Kamera, vor allem eine extern angeordnete zusätzliche Kamera, nicht erforderlich. Zudem muss die Kamera der endoskopischen Vorrichtung nicht speziell für das Erfassen der optischen Markierung ausgerichtet und/oder angeordnet werden, sondern die Erfassung findet im bereits für die spätere medizinische oder industrielle Untersuchung vorgesehene Anordnung und/oder Konfiguration statt. Folglich befindet sich die Kamera innerhalb der endoskopischen Vorrichtung bereits in der Anordnung und/oder Ausrichtung, welche für die Durchführung eines bestimmten medizinischen oder industriellen Eingriffes erforderlich ist. Folglich ist nach positiver Überprüfung der Identität die endoskopische Vorrichtung direkt einsatzbereit. Neben einer speziellen eingerichteten Kamera für das Erfassen eines Identifizierungscodes ist folglich auch kein externes und/oder zusätzliches Lesegerät zum Erkennen des Identifizierungscodes in der endoskopischen Vorrichtung notwendig. Dadurch weist die endoskopische Vorrichtung eine geringere Baugröße als bekannte Vorrichtungen nach dem Stand der Technik auf.

Des Weiteren ermöglicht das Erfassen durch die eigene Kamera der endoskopischen Vorrichtung ein schnelles Erkennen der Identität der Komponente und folglich einen schnellen Wechsel von einer Endoskopart auf eine andere, wenn dies beispielsweise unter einer Operation erforderlich sein kann. Dadurch ist sowohl ein Überprüfen der Identität des Endoskopes direkt nach der ersten Verbindung der Komponenten vor dem geplanten Eingriff als auch eine Überwachung und/oder ein Wechsel des Endoskops während des Eingriffes ermöglicht. Prinzipiell ist in jeder Einsatzsituation der endoskopischen Vorrichtung ein Erfassen und/oder ein Erkennen des sich jeweils in der Verwendung befindlichen Endoskops ermöglicht.

Zudem ist vorteilhafterweise auch eine Kamera aus einer früheren Baureihe mit einem Endoskop mit einem optischen Element und einer optischen Markierung in der endoskopischen Vorrichtung einsetzbar. Ebenso ist ein älteres Endoskop zur Verwendung in der endoskopischen Vorrichtung möglich, in dem ein Austausch eines bestehenden optischen Elementes mit einem neuen optischen Element mit einer optischen Markierung erfolgt, sodass in jedem Fall eine Identität des optischen Elementes und/oder des Endoskopes erfasst und erkannt werden kann. Folglich ist eine einfache Nachrüstbarkeit bereits bestehender Endoskope möglich, indem beispielsweise nur das Okular mit einer Markierung gegen ein altes Okular des Endoskopes ausgetauscht wird.

Einer der wesentlichen Gedanken der Erfindung beruht darauf, dass die endoskopische Vorrichtung als medizinisches System bereits mit seinen Komponenten für einen bestimmten medizinischen Eingriff konfiguriert ist und in dieser Konfiguration und/oder Anordnung mittels der eigenen Kamera zur Bildaufnahme des Objektfeldes innerhalb der endoskopischen Vorrichtung die optische Markierung eines optischen Elements einer Komponente und/oder des Endoskops erfasst und zur Identifizierung und/oder Verifizierung des medizinischen Systems zur Durchführung des bestimmten medizinischen Eingriffes genutzt wird.

### Folgendes Begriffliche sei erläutert:

Bei einer "endoskopischen Vorrichtung" handelt es sich insbesondere um ein medizinisches oder industrielles System zur endoskopischen Untersuchung mit mehreren Komponenten. Eine endoskopische Vorrichtung weist insbesondere mindestens eine Lichtquelle, einen Lichtleiter, eine Kamera und ein Endoskop selbst auf. Die endoskopische Vorrichtung wird für den jeweiligen Einsatz aus den dafür benötigten Komponenten zusammengesetzt, ausgerichtet und somit konfiguriert.

Eine "Lichtquelle" ist insbesondere ein Gerät, von dem Licht ausgeht, welches in das Endoskop eingekoppelt wird. Die Lichtquelle ist insbesondere extern angeordnet und über einen Lichtleiter mit dem Endoskop verbunden, wobei der Lichtleiter insbesondere über eine Steckverbindung am Endoskop weiter zu dem distalen Ende eines Schaftes des Endoskops geführt ist. Die Lichtquelle gewährleistet insbesondere eine homogene Ausleuchtung des Untersuchungsgebietes. Bei der Lichtquelle handelt es sich insbesondere um eine LED-Lichtquelle und/oder eine Kaltlichtquelle. Die Lichtquelle kann als eigenständiges Gerät ausgeführt sein oder direkt in einer Prozessoreinheit zur Bildverarbeitung integriert sein. Ebenso kann die Lichtquelle einen Luft-/CO₂-Insuflator aufweisen und mit diesem als ein Gerät ausgebildet sein. In diesem Fall weist die Lichtquelle auch die Insuflationspumpe auf. Ebenfalls können die Lichtquelle, Insuflationspumpe, Prozessoreinheit und/oder ein Monitor in einem einzigen Gerät ausgebildet sein. Die Lichtquelle ist insbesondere an die Art und/oder die optischen Eigenschaften der Kamera angepasst.

Ein "Lichtleiter" auch ("Lichtwellenleiter" genannt) ist insbesondere ein aus Lichtleitern bestehendes Kabel und/oder eine Leitung zur Übertragung von Licht. Ein Lichtleiter kann auch mittels eines Steckverbinders konfektioniert sein. Ein Lichtleiter weist insbesondere Fasern aus Quarzglas und/oder Kunststoff zum Führen des Lichtes auf. Bei einem Lichtleiter kann es sich insbesondere auch um einen Gellichtleiter oder einen Flüssigkeitslichtleiter handeln, welcher das Licht mittels eines Gels und/oder einer Flüssigkeit als Transportmedium leitet. Der Lichtleiter kann auch in einem Versorgungsschlauch integriert sein, welcher das Endoskop mit einem Medium oder weiteren Medien, wie beispielsweise einer Spülflüssigkeit, versorgt.

Eine "Kamera" ist insbesondere eine phototechnische Vorrichtung zur digitalen Wiedergabe der Inspektion. Dazu ist die Kamera über einen Kamerakopf insbesondere an dem Endoskop angeschlossen. Die digitale Wiedergabe kann hierbei über einen externen Monitor oder über einen direkt an und/oder in der Kamera integrierten Monitor erfolgen. Der Kamerakopf ist insbesondere über ein Okular mit dem Endoskop verbindbar. Somit bietet die Kamera insbesondere eine indirekte Sichtprüfung des Prüfbildes und/oder des Bildes des Objektfelds. Der Kamerakopf weist insbesondere eine HD-Auflösung auf. Die Kamera und/oder der Kamerakopf können insbesondere auch als Handgriff für den Benutzer dienen.

Ein "Endoskop" ist insbesondere ein Gerät, mit dem das Innere von Menschen oder Tieren oder technische Hohlräume untersucht und/oder manipuliert werden kann. Bei einem Endoskop handelt es sich insbesondere um ein starres Endoskop mit einem starren Schaft oder ein flexibles Endoskop mit einem flexiblen Schaft. Ein Endoskop ist insbesondere ein Videoendoskop mit einer digitalen Bilderzeugung und - übertragung zum proximalen Ende des Videoendoskops. Zumindest ein digitaler und/oder elektronischer Bildsensor ist insbesondere an einem distalen (benutzerfernen) Ende des langen Schafts des Endoskopes angeordnet. Ein Videoendoskop ist jede Art von digitalem Endoskop, zum Beispiel ein Laparoskop oder ein Gastroskop. Neben human- und tiermedizinischen Anwendungen kann ein Videoendoskop jedoch zu industriellen Zwecken, beispielsweise zur Sichtprüfung in schwer zugänglichen Hohlräumen, eingesetzt werden. Bei industriellen Anwendungen wird ein Endoskop häufig auch als Boroskop bezeichnet.

Ein "optisches Element" ist insbesondere ein Element zum Durchlassen, Formen und/oder Verändern eines Lichtstrahls. Das optische Element ist insbesondere im Strahlengang des Endoskops angeordnet und/oder entlang der optischen Achse. Die optische Achse ist insbesondere eine imaginäre Linie, welche den Weg definiert, auf dem sich Licht durch das Endoskop und/oder seinem Objektivlinsensystem bis zum distalen Ende und/oder zum elektronischen Bildsensor ausbreitet. Bevorzugt verläuft die optische Achse durch den Mittelpunkt des optischen Elementes. Das optische Element weist insbesondere Glas und/oder Kunststoff auf. Bei einem optischen Element kann es sich beispielsweise um eine Linse oder ein Okular handeln.

Bei einer "optischen Markierung" handelt es sich insbesondere um eine auf-, ein- und/oder angebrachte Markierung auf und/oder in einem optischen Element. Eine optische Markierung stellt insbesondere einen Identifikationscode dar, welcher mittels der Kamera erfasst und/oder erkannt wird. Somit beinhaltet die optische Markierung Informationen zur Identität des optischen Elementes und/oder des Endoskopes. Bevorzugt ist die optische Markierung an einem optischen Element angebracht, welches im Inneren des Endoskops angeordnet ist, sodass die optische Markierung nicht entfernbar und/oder manipulierbar ist. Jedoch kann die optische Markierung auch an der Außenseite des Endoskops angeordnet sein, beispielsweise auf der Innenseite der Okularmuschel, welche zur Kamera ausgerichtet ist.

In einer weiteren Ausführungsform der endoskopischen Vorrichtung weisen zwei optische Elemente, drei optische Elemente und/oder mehrere optische Elemente jeweils eine optische Markierung auf.

Dadurch können mehrere Bestandteile des Endoskops, welche zur Ausbildung des Endoskops beispielsweise verbunden werden, wie das Okular und der Abschnitt mit dem Schaft, jeweils optisch markiert und identifiziert werden. Ebenso kann aber auch ein einziges optisches Element redundant mit zwei oder mehreren optischen Markierungen versehen sein. Ebenso können mehrere entlang der optischen Achse angeordneten Linsen eines Objektivsystems jeweils eine optische Markierung aufweisen und somit eine Redundanz in der Identifizierung gewährleisten.

Bei den zweiten, dritten und/oder mehreren optischen Elementen handelt es sich in der jeweiligen Funktion und Ausführung um ein oben definiertes optisches Element.

Um das Erfassen und/oder Bestimmen der Identität an die Art des Endoskops, seiner Bestandteile und/oder der optischen Anforderung und der Art des Einsatzzweckes anzupassen, ist das optische Element und/oder sind die optischen Elemente ein Okular, eine Linse, insbesondere eine Stablinse, und/oder ein Deckglas.

Somit kann das optische Element, welches die optische Markierung aufweist, gezielt aus einer Vielzahl von möglichen optischen Elementen vom proximalen Ende, beispielsweise dem Okular, bis zum distalen Ende, dem Deckglas, des Endoskops ausgewählt werden. Zum Beispiel kann sich die Seriennummer als optische Markierung in Klartext, als Barcode oder QR-Code auf der innenliegenden Deckglasseite im Deckglas des Endoskops befinden. Es ist besonders vorteilhaft, dass entlang der gesamten Längsabmessung des Endoskops ein optisches Element oder mehrere optische Elemente mit einer jeweiligen optischen Markierung versehen sind, welche innenliegend, fest verbunden und/oder örtlich unbeweglich im Inneren des Endoskops angeordnet sind. Dadurch unterliegt die jeweilige optische Markierung weder Abnutzungserscheinungen und Verschleiß, wie beispielsweise durch die Reinigung und Desinfektion, noch ist eine äußere Manipulation der optischen Markierung möglich.

Ein "Okular" ist insbesondere ein augenseitig und/oder kameraseitig optisch wirksamer Teil eines optischen Systems. Ein Okular weist insbesondere eine einzelne Linse oder ein Linsensystem auf. Ein Okular ist insbesondere ein Teil des Endoskopes, mittels welchem mit dem Auge zum Objektfeld und/oder der Prüfstelle geblickt werden kann. Zur digitalen Wiedergabe der Inspektion kann das Endoskop über das Okular mit dem Kamerakopf der Kamera verbunden sein.

Eine "Linse" ist insbesondere ein durchlässiger optischer Körper, der einen Lichtstrahl oder mehrere Lichtstrahlen mittels Brechung fokussiert oder streut. Bei einer Linse handelt es sich insbesondere um eine sphärische oder asphärische Linse.

Eine "Stablinse" ist insbesondere eine Zylinderlinse, die am Umfang poliert und an beiden Enden geschliffen ist. Eine Stablinse kann insbesondre zur Lichtleiterkopplung verwendet werden.

Ein "Deckglas" ist insbesondere ein planares, rundes Glas, welches das distale Ende des rohrförmigen Schaftes verschließt. Das Deckglas ist insbesondere flüssigkeits- und gasdicht mit dem distalen Ende des Schaftes verbunden, beispielsweise durch Verkleben, Umspritzen mit Kunststoff oder Verglasen.

Erfindungsgemäß weist die Kamera ein Zoomobjektiv mit einem Verstellmittel zum Verändern einer Brennweite derart auf, dass mit dem Zoomobjektiv die optische Markierung oder die optischen Markierungen in einem Bereich von einem proximalen Ende bis zu einem distalen Ende des Endoskops jeweils in einer definierten Markierungszoomstellung erfassbar ist oder sind.

Somit fokussiert die Kamera mittels des Zoomobjektivs so weit, dass diese sowohl im Deckglas oder im Linsensystem angebrachte optische Markierungen erfasst und/oder erkennt, aber auch am gegenüberliegenden proximalen Ende des Endoskops eine beispielsweise eingebrachte Seriennummer im Okular erfasst. Folglich kann die Kamera vollständig bis in das Endoskop von dessen proximalen Ende (benutzernahen) bis zum distalen (benutzerfernen) Ende fokussieren. Es ist besonders vorteilhaft, dass die Kamera in jeweils definierten Markierungszoomstellungen und somit Fokussierungen die jeweilige optische Markierung eines optischen Elementes erfasst und sichtbar macht.

Ein "Zoomobjektiv" (auch kurz "Zoom" genannt) ist insbesondere ein Objektiv mit einer variablen Brennweite. Mittels des Verstellmittels des Zoomobjektivs wird insbesondere durch ein Verschieben von Linsenelementen im Objektiv eine Veränderung der Brennweite bewirkt. Hierzu kann das Verstellmittel insbesondere manuell, beispielsweise durch Drehen oder Verschieben eines Rings am Objektiv, oder motorisch, durch Drücken einer Zoomtaste, betätigt werden.

Eine "definierte Markierungszoomstellung" ist insbesondere eine definierte Einstellung des Zoomobjektivs, bei der die optische Markierung erfassbar und/oder sichtbar ist.

Damit die Sicht auf das Objektfeld und/oder die Bildaufnahme frei von einer optischen Markierung ist, ist das Zoomobjektiv mittels des Verstellmittels derart einstellbar, dass in einer Objektzoomstellung zum Betrachten eines zu untersuchenden Objektes ein Bild der Kamera in der Objektzoomstellung frei von der optischen Markierung oder den optischen Markierungen ist.

Somit ist die optische Markierung beim normalen Arbeiten und bei der vorgesehenen Verwendung des Endoskops für die Durchführung eines bestimmten medizinischen Eingriffs oder einer industriellen Untersuchung nicht für den Benutzer, auf der Bildaufnahme und/oder den Monitor sichtbar.

Dadurch kann zeitnah zuerst eine Fokussierung auf eine optische Markierung, beispielsweise im Deckglas des Endoskopes, mittels des Zoomobjektives und des Verstellmittels in der definierten Markierungszoomstellung erfolgen und direkt anschließend nach Identifizierung des Deckglases wird die Fokussierung zum Erreichen einer definierten Objektzoomstellung fortgesetzt, sodass die optische Markierung unsichtbar und das zu untersuchende Objekt optimal sichtbar ist. Dadurch wird eine Störung durch die optische Markierung bei Betrachten des zu untersuchenden Objektes vermieden.

Zudem kann sehr schnell und effizient durch die Fokussierung auf eine definierte Markierungszoomstellung und eine definierte Objektzoomstellung zwischen einem Modus zur Bestimmung der Identität und einem konventionellen Untersuchungsmodus gewechselt werden.

Eine "Objektzoomstellung" ist insbesondere eine mittels des Verstellmittels eingestellte Zoomstellung des Zoomobjektivs, in der das zu untersuchende Objekt und/oder das aufgenommene Bild der Kamera frei von einer optischen Markierung ist. Somit ist das zu untersuchende Objektfeld in der Objektzoomstellung optimal sichtbar und/oder vollständig ausgeleuchtet.

In einer weiteren Ausführungsform der endoskopischen Vorrichtung ist die optische Markierung und/oder sind die optischen Markierungen eine Strichmarkierung, eine Farbmarkierung, ein QR-Code, eine Seriennummer und/oder ein Klartext.

Somit kann für jedes optische Element die jeweils am besten geeignete optische Markierung verwendet werden, wobei alle Markierungen mittels der Kamera prinzipiell erfassbar sind.

Dadurch erkennt die Kamera beispielsweise eine Seriennummer im optischen Linsensystem im Schaft des Endoskopes bei einer bestimmten Fokussierung als auch die Seriennummer im Okular des Endoskopes.

Zudem können durch verschiedene optische Markierungen, beispielsweise einer Seriennummer, eines QR-Codes und eines Strichcodes, an verschiedenen optischen Elementen des Endoskops ebenfalls eine Redundanz in der Erfassung der Identität geschaffen werden.

Eine "Strichmarkierung" ist insbesondere eine optoelektronisch lesbare Schrift, welche aus verschieden breiten oder gleich breiten, parallelen Strichen und/oder Lücken zwischen den Strichen besteht. Die Strichmarkierung bildet insbesondere die Identitätsdaten in binären Symbolen ab. Bei einer Strichmarkierung kann es sich insbesondere um einen Strichode, Balkencode, Streifencode oder Barcode handeln. Die Identitätsdaten in der Strichmarkierung werden insbesondere mit der Kamera als optisches Lesegerät maschinell eingelesen und elektronisch weitergegeben und/oder weiterverarbeitet.

Eine "Farbmarkierung" ist insbesondere eine optische Markierung, welche eine bestimmte Farbe oder mehrere Farben aufweist. Die Farbmarkierung weist insbesondere eine Kennfarbe auf, welche einer Identität zugeordnet werden kann. Eine Farbmarkierung kann auch einen bestimmten Farbstoff aufweisen, welcher nur bei Bestrahlung mit bestimmtem Licht, wie beispielsweise UV- oder IR-Licht, sichtbar ist.

Ein "QR-Code" ist insbesondere eine optische Markierung, welche einen zweidimensionalen Code aufweist. Der QR-Code besteht insbesondere aus einer quadratischen Matrix aus schwarzen und weißen Quadraten, die die codierten Identitätsdaten binär darstellen. Im QR-Code wird mittels einer speziellen Markierung in drei der vier Ecken des Quadrates insbesondere eine Orientierung vorgegeben. Mit der Kamera wird insbesondere ein digitales Bild der QR-codierten Daten erzeugt und/oder anschließend die im Bild enthaltenen codierten Identifikationsdaten in Textform umgewandelt und somit decodiert.

Eine "Seriennummer" ist insbesondere eine eindeutige Bezeichnung eines Produktes durch den Hersteller. Die Seriennummer bezeichnet insbesondere das optische Element, Bestandteile des Endoskops und/oder das Endoskop. Die Seriennummer stellt insbesondere auch einen Identifikator für die Bauteile und/oder das Endoskop einer Serie dar und/oder erlaubt eine Rückverfolgung auf die Produktion dieser.

Zum optimalen Auf- und/oder Anbringen der optischen Markierung in Abhängigkeit des Materials des jeweiligen optischen Elementes und/oder der optischen Eigenschaften des Endoskops, ist die optische Markierung in und/oder auf das optische Element oder den optischen Elementen geätzt, graviert, gelasert, aufgedruckt und/oder geschrieben.

In einer weiteren Ausführungsform der endoskopischen Vorrichtung ist die optische Markierung in der Mitte, am äußeren Rand und/oder quer über eine Oberfläche des optischen Elementes oder der optischen Elemente angeordnet.

Dadurch kann je nach Form des jeweiligen optischen Elementes und/oder dessen Anordnung innerhalb des Strahlenganges und/oder entlang der optischen Achse eine optimale Position der Markierung gewählt werden. Zudem kann dadurch zusätzlich eine Differenzierung zwischen dem Modus der sichtbaren optischen Markierung zum Identifizieren und dem Modus des optimal sichtbaren Objektfeldes zur Untersuchung erfolgen.

Damit die optische Markierung nur unter definierten Lichtverhältnissen sichtbar ist, kann die optische Markierung einen Farbstoff, insbesondere einen UV- und/oder Fluoreszenzfarbstoff, aufweisen.

Dadurch kann beispielsweise eine optische Markierung mit einem Fluoreszenzfarbstoff so gewählt werden, dass die Emissionswellenlänge des Fluoreszenzfarbstoffs bei einer Beobachtung des Objektfeldes im sichtbaren und/oder Weißlichtbereich nicht stört. Ebenso kann bei einer photodynamischen Diagnostik die Fluoreszenzemission eines diagnostisch eingesetzten Fluoreszenzfarbstoffes ungestört betrachtet werden, wenn die optische Markierung mit einem Farbstoff ausgeführt ist, die in diesem Wellenbereich weder Licht absorbiert noch emittiert. Somit kann durch gezielte Wahl eines Farbstoffes für die Markierung auch frei von der definierten Markierungszoomstellung und/oder der definierten Objektzoomstellung eine Sichtbarkeit und Unsichtbarkeit der optischen Markierung alleine über die jeweiligen Lichteigenschaften erzielt werden.

Ein "Fluoreszenzfarbstoff" ist insbesondere ein Farbstoff, welcher bei Anregung von Licht mit einer kürzeren Wellenlänge Fluoreszenzlicht mit einer höheren Wellenlänge emittiert.

Ein "UV-Farbstoff" (auch "photochromer Farbstoff" genannt) ist insbesondere ein Farbstoff, welcher auf Bestrahlung mit UV-Licht mit einer reversiblen Farbtonänderung reagiert. Bei einem UV-Farbstoff verändert das eingestrahlte UV-Licht insbesondere die chemische Struktur des UV-Farbstoffs und ändert damit sein Absorptionsverhalten. Beispielsweise wechselt der UV-Farbstoff von weiß auf violett, blau, gelb und/oder rot.

In einer weiteren Ausführungsform ist der endoskopischen Vorrichtung eine Bild- und/oder Datenverarbeitungseinrichtung zum Auswerten und Verarbeiten eines aufgenommenen Bildes und/oder Identifizieren der jeweiligen optischen Markierung des jeweiligen optischen Elementes und/oder der optischen Elemente zugeordnet oder die endoskopische Vorrichtung weist die Bild- und/oder Datenverarbeitungseinrichtung auf.

Nach Auswerten und Verarbeiten der aufgenommenen Bilddaten mittels der Bild- und/oder Datenverarbeitungseinrichtung und der eindeutigen Identifizierung des jeweiligen optischen Elementes und/oder des Endoskops kann mittels der Bild- und/oder Datenverarbeitungseinrichtung die Konfiguration der endoskopischen Vorrichtung für den geplanten medizinischen Eingriff freigegeben und/oder blockiert werden. Dazu erfolgt eine entsprechende Ausgabe an den Benutzer und/oder es wird direkt eine Funktion der endoskopischen Vorrichtung freigegeben oder blockiert. Beispielsweise kann bei einem für den PDD- oder ICG-Modus ungeeignetes Endoskop direkt über die Bild- und/oder Datenverarbeitungseinrichtung ein Einkoppeln von Licht in dieses Endoskop unterbrochen werden.

Ebenso kann bei einer ausgelieferten endoskopischen Vorrichtung mit mehreren Komponenten nur ein Betrieb ermöglicht werden, wenn mittels der Kamera die vorgegebenen optischen Markierungen erfasst und mittels der Bild- und/oder Datenverarbeitungseinrichtung ausgewertet und richtig identifiziert worden sind.

Die Bild- und/oder Datenverarbeitungseinrichtung kann zudem die Kamerakontrolleinheit und/oder die bereits oben beschriebene Prozessoreinheit aufweisen. Zudem kann die Bild- und/oder Datenverarbeitungseinrichtung auch alle oder nur einige Funktionen der endoskopischen Vorrichtung steuern und/oder regeln. Selbstverständlich kann die Bild- und/oder Datenverarbeitungseirichtung auch direkt in der Kamera integriert sein. Die Bild- und/oder Datenverarbeitungseinrichtung weist insbesondere eine Software auf, welche direkt die optische Markierung erkennt, eine Reaktion in Abhängigkeit von dem Identifikationsergebnis abhängige Reaktion und/oder eine Benachrichtigung an den Anwender, wie beispielsweise die Anzeige einer Warnung, herausgibt.

In einem zusätzlichen Aspekt der Erfindung wird die Aufgabe gelöst durch ein Verfahren zur Überprüfung einer Identität einer Komponente einer endoskopischen Vorrichtung nach einem der Ansprüche 1-9 mit folgenden Schritten:
- Konfigurieren der endoskopischen Vorrichtung durch Anordnen und/oder Verbinden der Lichtquelle, des Lichtleiters, der Kamera und des Endoskops aufweisend ein optisches Element oder mehrere optische Elemente,
- Fokussieren der Kamera auf mindestens eine optische Markierung eines der optischen Elemente, wobei das Zoomobjektiv die optische Markierung oder die optischen Markierungen in einem Bereich von einem proximalen Ende bis zu einem distalen Ende des Endoskops jeweils in einer definierten Markierungszoomeinstellung erfasst,
- Aufnehmen eines Bildes der mindestens einen optischen Markierung mittels der Kamera,
- Analysieren des aufgenommenen Bildes und Identifizieren der mindestens einen optischen Markierung und/oder des optischen Elementes des Endoskopes,
- Wiederholen des Fokussierens, Aufnehmens, Analysierens und Identifizierens einer weiteren optischen Markierung eines weiteren optischen Elementes und/oder Ausgeben eines Ergebnisses des Identifizierens für eine medizinische oder industrielle Anwendung der konfigurierten endoskopischen Vorrichtung.

Somit ist mittels des Verfahrens eine Überprüfung einer Identität einer Komponente der endoskopischen Vorrichtung und somit die optimale Konfiguration dieser Vorrichtung für einen bestimmten vorgesehenen Einsatz, direkt vor diesem Einsatz durchzuführen, ohne dass die endoskopische Vorrichtung für das Überprüfen der Identität oder das anschließende Untersuchen des Objektes bei einem positiven Ergebnis des Identifizierens umgebaut und/oder anders angeordnet werden muss. Folglich kann nach erfolgreichem Identifizieren direkt ohne Zeitverlust mit dem geplanten medizinischen Eingriff oder der industriellen Anwendung begonnen werden.

In einer weiteren Ausgestaltungsform des Verfahrens ist beim Fokussieren der Kamera auf ein zu untersuchendes Objekt die optische Markierung oder sind die optischen Markierungen nicht sichtbar.

Um nach einem Verbinden und somit Konfigurieren der Komponenten der endoskopischen Vorrichtung direkt das Verfahren zum Überprüfen einer Identität einer Komponente automatisch durchzuführen, wird das Fokussieren der Kamera automatisch bei einem Verbinden mit dem Endoskop oder beim Einschalten des Endoskopes durchgeführt.

Somit wird das Verfahren automatisch gestartet und muss nicht manuell durch einen Benutzer ausgelöst werden. Für das automatische Identifizieren erkennt die Kamera beim Aufstecken des Endoskops auf den Kamerakopf aufgrund von veränderten Lichtverhältnissen oder einem Magnetschalter, dass ein Endoskop aufgesteckt worden ist. Die Kamera fährt anschließend automatisch das Zoomobjektiv in die vorgesehene Markierungszoomstellung, um ein Erkennen beispielsweise einer Seriennummer als optische Markierung durchzuführen. Bei einem positiven Ergebnis der Identifizierung werden anschließend automatisch die entsprechenden Funktionen der endoskopischen Vorrichtung für den geplanten Einsatz freigeschaltet.

In einem anderen Aspekt wird die Aufgabe gelöst durch ein Computerprogrammprodukt gemäß Anspruch 13 zum Auswerten einer Bildaufnahme einer optischen Markierung eines optischen Elements und zum Identifizieren einer oben beschriebenen endoskopischen Vorrichtung nach Anspruch 9, wobei das Computerprogrammprodukt Befehle aufweist, welche bei der Ausführung des Computerprogrammproduktes durch die Bild- und/oder Datenverarbeitungseinrichtung ein Erkennen der optischen Markierung in der Bildaufnahme, ein Zuordnen der erkannten, optischen Markierung zu einem bestimmten optischen Element und/oder zu einem bestimmten Endoskop und somit ein Identifizieren des bestimmten optischen Elementes und/oder des bestimmten optischen Endoskops und/oder ein Ausgeben einer Benachrichtigung an den Benutzer der endoskopischen Vorrichtung ausführt.

Im Weiteren wird die Erfindung anhand von Ausführungsbeispielen näher erläutert. Es zeigen
- Figur 1: eine stark schematische Darstellung einer endoskopischen Vorrichtung mit einer Kamera und einem Endoskop ausgerichtet auf ein Gewebe,
- Figur 2: eine Bildaufnahme der Kamera bei Durchblick durch das Endoskop mit jeweils scharf gestellten Strichcode, QR-Code und Seriennummer und unscharfer Darstellung des Gewebes,
- Figur 3: eine Bildaufnahme der Kamera bei Durchblick durch das Endoskop mit einer Fokussierung auf das sichtbare Gewebe frei von den Codes aus Figur 2,
- Figur 4: eine Darstellung des Verfahrens zur Konfigurations-/Identitätsüberprüfung, und
- Figur 5: eine dreidimensionale Darstellung der endoskopischen Vorrichtung.

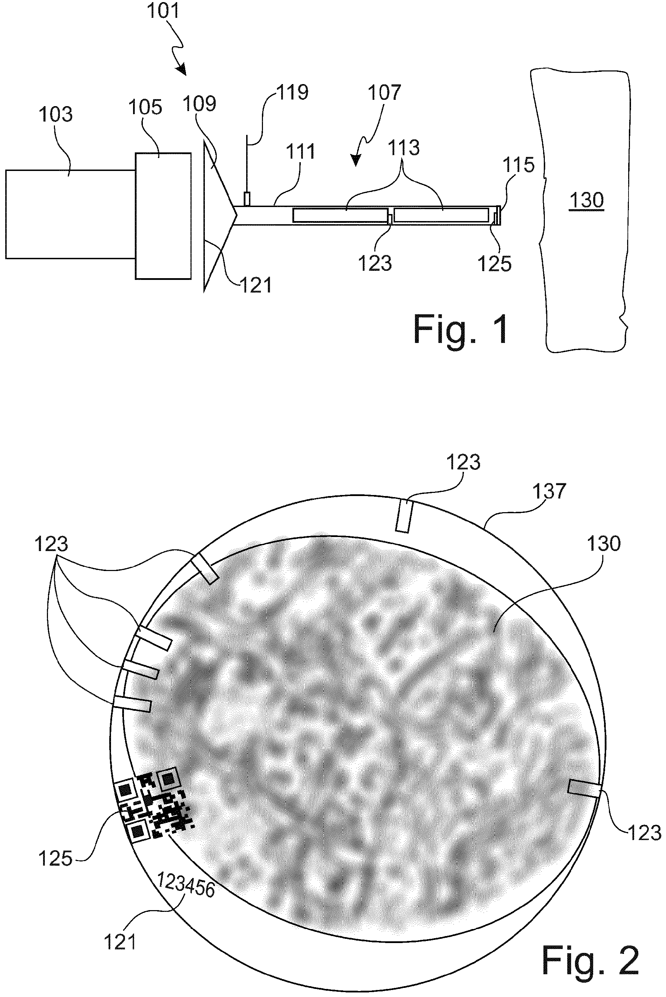

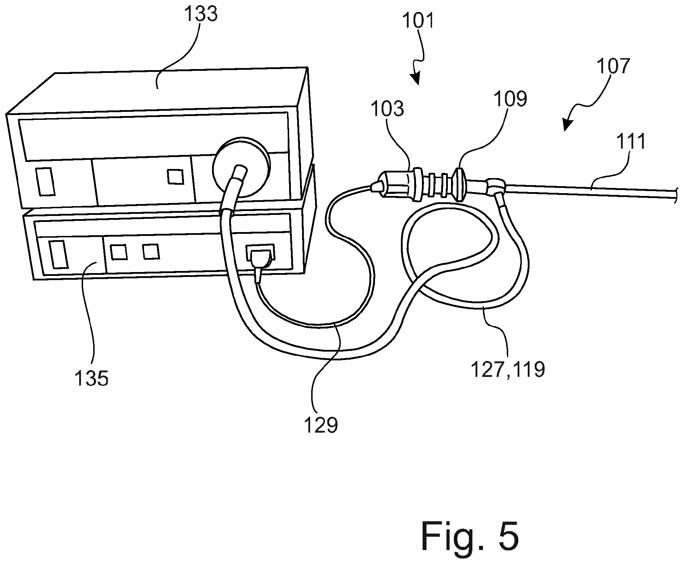

Eine endoskopischen Vorrichtung 101 weist eine Kamera 103 mit einem Kamerazoom 105, eine zur Kamera 103 angepasste Lichtquelle 133 und ein Endoskop 107 auf (Fig. 1 und 5). In distaler Richtung folgt nach der Kamera 103 und dem Kamerazoom 105 ein Okular 109 des Endoskops 107. Das Endoskop 107 weist neben dem Okular 109 einen langgestreckten Schaft 111 auf.

Mit dem Endoskop 107 ist über einen Lichtleiteranschluss ein Lichtleiter 119 geführt in einem Versorgungsschlauch 127 und die Kamera 103 über ein Videokabel 129 mit einer Bild- und Datenverarbeitungseinrichtung 135 verbunden (Fig. 5). Der Lichtleiter 119 ist mit der Lichtquelle 133 zum Einspeisen von Licht in den Lichtleiter 119 verbunden. Im Inneren innerhalb des Schaftes 111 weist das Endoskop 107 zwei Stablinsen 113 auf. Der Schaft 111 ist am distalen Ende mittels eines Deckglases 115 verschlossen. Das Okular 109 weist eine Seriennummer 121 als Identifikationscode auf. Des Weiteren weist in distaler Richtung die erste Stablinse 113 an ihrer distalen Seite einen Strichcode 123 auf und das Deckglas 115 weist an seiner proximal gelegenen Seite einen QR-Code 125 auf. Die endoskopische Vorrichtung 101 ist bereits vollständig für den geplanten Einsatz zur Untersuchung eines Gewebes 130 konfiguriert (Figur 1).

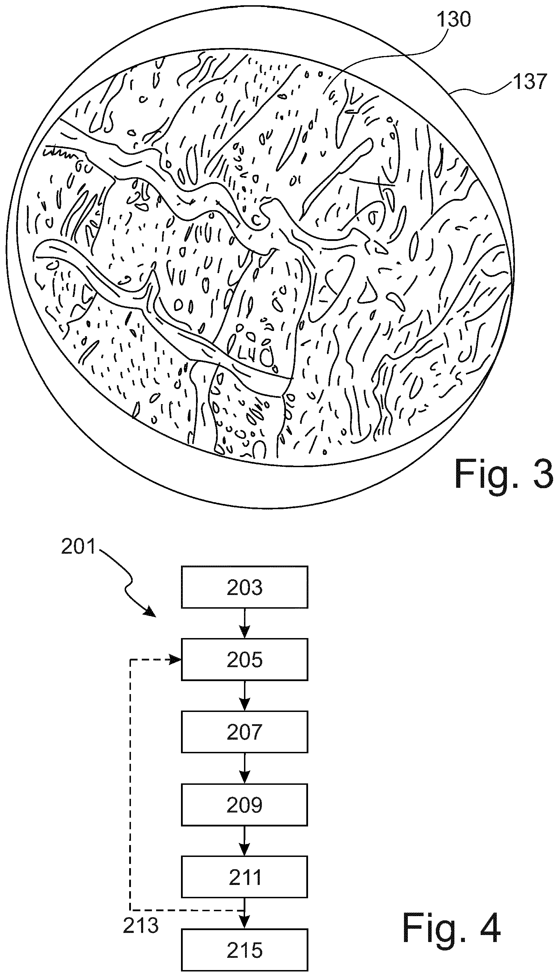

Mittels des Kamerazooms 105 wird nun die Kamera auf eine erste Markierungszoomstellung zum Erfassen der Seriennummer 121 des Okulars 109 fokussiert, die Seriennummer 121 am Rande des Okulars 109 erfasst und mittels der Bild- und Datenverarbeitungseinrichtung 135 werden die Bilddaten ausgewertet und die Seriennummer 121 identifiziert. Anschließend fokussiert die Kamera 103 mittels des Kamerazooms 105 auf eine zweite Markierungszoomstellung zum Erfassen des Strichcodes 123 an der distalen Seite der ersten Stablinse 113. Die entsprechend aufgenommenen Bilddaten werden ebenfalls wie bei der Seriennummer 121 auch für den Strichcode 123 ausgewertet und identifiziert. Im nächsten Schritt erfolgt mittels des Kamerazooms 105 eine Fokussierung auf eine dritte Markierungszoomstellung zum Erfassen des QR-Codes 125 auf der proximalen Seite des Deckglases 115. Die Auswertung und Identifizierung erfolgt für den QR-Code 125 äquivalent (hierzu wird herausgestellt, dass in Figur 3 alle drei nacheinander angefahrenen Markierungszoomstellungen mit der entsprechenden Bildaufnahme des jeweiligen Codes im Sichtfeld 137 gemeinsam dargestellt sind, aber vom Benutzer nacheinander gesehen werden).

Die Identifizierung der Seriennummer 121 des Okulars 109, des Strichcodes 123 der ersten Stablinse 113 und des QR-Codes 125 des Deckglases 115 ergibt, dass das Endoskop 107 optimal für den geplanten endoskopischen Eingriff am Gewebe 130 geeignet und zur Verwendung mit der Kamera 103 und der Lichtquelle 133 passend ist. Daraufhin wird unmittelbar mit der Untersuchung des Gewebes 130 durch einen Benutzer begonnen, welcher das distale Ende des Schafts 111 des Endoskops 107 an das Gewebe 130 heranführt. Hierbei ist das Gewebe 130 für den Benutzer scharf sichtbar, ohne dass die Seriennummer 121, der Strichcode 123 und der QR-Code 125 in der Bildaufnahme im Sichtfeld 137 sichtbar sind (siehe Figur 3).

Folglich wird ein Verfahren zur Konfigurations-/ Identitätsprüfung 201 bereitgestellt, bei dem zunächst ein Konfigurieren 203 der endoskopischen Vorrichtung 101 für einen geplanten, bestimmten endoskopischen Einsatz erfolgt. Anschließend wird ein Fokussieren 205 der Kamera 103 mittels des Kamerazooms 105 durchgeführt, bei dem in einer definierten Markierungszoomstellung eine Bildaufnahme 207 einer optischen Markierung 121, 123 oder 125 mittels der Kamera 103 erfolgt. Im nächsten Schritt wird das aufgenommene Bild 209 mittels einer Bild- und/oder Datenverarbeitungseinrichtung analysiert (Schritt 209) und es erfolgt ein Identifizieren 211 der optischen Markierung. Bei weiteren optischen Markierungen in der endoskopischen Vorrichtung 101 erfolgt ein Wiederholen 213 der Schritte 205 bis 211 oder das Ergebnis des Identifizierens 211 wird an den Benutzer ausgegeben (Schritt 215). Das Ausgeben 215 an den Benutzer kann in Form eines Warnsignals, einer Anzeige und/oder eines Freigebens oder Blockierens einer Funktion der endoskopischen Vorrichtung 101 erfolgen.

Somit wird eine endoskopische Vorrichtung 101 bereitgestellt, bei welcher direkt mittels der bildaufnehmenden Kamera 103 vor dem eigentlich geplanten Einsatz der bereits konfigurierten endoskopischen Vorrichtung 101, die Identität des Endoskopes 107 zeitnah überprüft und bei positivem Identifizieren direkt die endoskopische Vorrichtung 101 für den nun durchzuführenden Einsatz freigegeben. Folglich wird mit derselben endoskopischen Vorrichtung 101 sowohl die Identität der einzelnen Bestandteile des Endoskops 107 und somit des Endoskops 107 selbst, als auch die vorgegebene Untersuchung des Gewebes 130 ermöglicht.

### Bezugszeichenliste

- 101: Endoskopische Vorrichtung
- 103: Kamera
- 105: Kamerazoom
- 107: Endoskop
- 109: Okular
- 111: Schaft
- 113: Stablinse
- 115: Deckglas
- 119: Lichtleiter
- 121: Seriennummer
- 123: Strichcode
- 125: QR-Code
- 127: Versorgungsschlauch
- 129: Videokabel
- 130: Gewebe
- 133: Lichtquelle
- 135: Bild- und Datenverarbeitungseinrichtung
- 137: Sichtfeld
- 201: Verfahren zur Konfigurations-/Identitätsüberprüfung
- 203: Konfigurieren
- 205: Fokussieren der Kamera
- 207: Bildaufnehmen einer optischen Markierung
- 209: Analysieren des Bildes
- 211: Identifizieren
- 213: Wiederholen mit weiterer optischer Markierung
- 215: Ausgeben an Benutzer

## Patentansprüche

1. Endoskopische Vorrichtung (101), insbesondere medizinische oder industrielle endoskopische Vorrichtung, wobei die endoskopische Vorrichtung (101) als Komponenten eine Lichtquelle, einen Lichtleiter (119), eine Kamera (103) und ein Endoskop (107) mit einem optischen Element (109, 113, 115) oder mehreren optischen Elementen aufweist, wobei mindestens ein optisches Element (109, 113, 115) eine optische Markierung (121, 123, 125) zur Überprüfung einer Identität des optischen Elementes (109, 113, 115) und/oder des Endoskopes (107) derart aufweist, dass die optische Markierung (121, 123, 125) mit der Kamera (103) zur Überprüfung der Identität und/oder einer Konfiguration der endoskopischen Vorrichtung (101) erfassbar ist, **dadurch gekennzeichnet, dass** die Kamera (103) ein Zoomobjektiv (105) mit einem Verstellmittel zum Verändern einer Brennweite derart aufweist, dass mit dem Zoomobjektiv (105) die optische Markierung oder die optischen Markierungen (121, 123, 125) in einem Bereich von einem proximalen Ende bis zu einem distalen Ende des Endoskops (107) jeweils in einer definierten Markierungszoomstellung erfassbar ist oder sind.

2. Endoskopische Vorrichtung (101) nach Anspruch 1, **dadurch gekennzeichnet, dass** zwei optische Elemente, drei optische Elemente (109, 113, 115) und/oder mehrere optischen Elemente jeweils eine optische Markierung aufweisen.

3. Endoskopische Vorrichtung (101) nach Anspruch 1 oder 2, **dadurch gekennzeichnet, dass** das optische Element und/oder die optischen Elemente ein Okular (109), eine Linse, insbesondere eine Stablinse (113), und/oder ein Deckglas (115) ist oder sind.

4. Endoskopische Vorrichtung (101) nach einem der vorherigen Ansprüche, **dadurch gekennzeichnet, dass** das Zoomobjektiv (105) mittels des Verstellmittels derart einstellbar ist, dass in einer Objektzoomstellung zum Betrachten eines zu untersuchenden Objektes (130) ein Bild der Kamera (103) in der Objektzoomstellung frei von der optischen Markierung oder den optischen Markierungen (121, 123, 125) ist.

5. Endoskopische Vorrichtung (101) nach einem der vorherigen Ansprüche, **dadurch gekennzeichnet, dass** die optische Markierung und/oder die optischen Markierungen eine Strichmarkierung (123), eine Farbmarkierung, ein QR-Code (125), eine Seriennummer (121) und/oder ein Klartext ist oder sind.

6. Endoskopische Vorrichtung (101) nach einem der vorherigen Ansprüche, **dadurch gekennzeichnet, dass** die optische Markierung (121, 123, 125) in und/oder auf das optische Element oder den optischen Elementen (109, 113, 115) geätzt, graviert, gelasert, aufgedruckt und/oder geschrieben ist.

7. Endoskopische Vorrichtung (101) nach einem der vorherigen Ansprüche, **dadurch gekennzeichnet, dass** die optische Markierung (121, 123, 125) in der Mitte, am äußeren Rand und/oder quer über eine Oberfläche des optischen Elementes oder der optischen Elemente (109, 113, 115) angeordnet ist.

8. Endoskopische Vorrichtung (101) nach einem der vorherigen Ansprüche, **dadurch gekennzeichnet, dass** die optische Markierung (121, 123, 125) einen Farbstoff, insbesondere einen UV- und/oder Fluoreszenzfarbstoff, aufweist.

9. Endoskopische Vorrichtung (101) nach einem der vorherigen Ansprüche, **dadurch gekennzeichnet, dass** der endoskopischen Vorrichtung (101) eine Bild- und/oder Datenverarbeitungseinrichtung zum Auswerten und Verarbeiten eines aufgenommenen Bildes und/oder Identifizieren der jeweiligen optischen Markierung (121, 123, 125) des optischen Elementes und/oder der optischen Elemente (109, 113, 115) zugeordnet ist oder die endoskopische Vorrichtung (101) die Bild- und/oder Datenverarbeitungseinrichtung aufweist.

10. Verfahren (201) zur Überprüfung einer Identität einer Komponente einer endoskopischen Vorrichtung (101) nach einem der Ansprüche 1 - 9 mit folgenden Schritten:
- Konfigurieren (203) der endoskopischen Vorrichtung (101) durch Anordnen und/oder Verbinden der Lichtquelle, des Lichtleiters (119), der Kamera (103) und des Endoskops (107) aufweisend ein optisches Element oder mehrere optische Elemente (109, 113, 115),
- Fokussieren (205) der Kamera (103) auf mindestens eine optische Markierung (121, 123, 125) eines der optischen Elemente (109, 113, 115), wobei das Zoomobjektiv (105) die optische Markierung oder die optischen Markierungen (121, 123, 125) in einem Bereich von einem proximalen Ende bis zu einem distalen Ende des Endoskops (107) jeweils in einer definierten Markierungszoomstellung erfasst,
- Aufnehmen (207) eines Bildes der mindestens einen optischen Markierung (121, 123, 125) mittels der Kamera (103),
- Analysieren (209) des aufgenommenen Bildes und Identifizieren (211) der mindestens einen optischen Markierung (121, 123, 125) und/oder des optischen Elementes (109, 113, 115) des Endoskopes (107),
- Wiederholen (213) des Fokussierens (205), Aufnehmens (207), Analysierens (209) und Identifizierens (211) einer weiteren optischen Markierung (121, 123, 125) eines weiteren optischen Elementes (109, 113, 115) und/oder Ausgeben (215) eines Ergebnisses des Identifizierens (211) für eine medizinische oder industrielle Anwendung der konfigurierten endoskopischen Vorrichtung (101).

11. Verfahren (201) nach Anspruch 10, **dadurch gekennzeichnet, dass** beim Fokussieren (205) der Kamera (103) auf ein zu untersuchendes Objekt (130) die optische Markierung oder optischen Markierungen (121, 123, 125) nicht sichtbar ist oder sind.

12. Verfahren (201) nach einem der Ansprüche 10 oder 11, **dadurch gekennzeichnet, dass** das Fokussieren (205) der Kamera (103) automatisch bei einem Verbinden mit dem Endoskop (107) oder beim Einschalten des Endoskopes (107) durchgeführt wird.

13. Computerprogrammprodukt zum Auswerten einer Bildaufnahme einer optischen Markierung (121, 123, 125) eines optischen Elementes (109, 113, 115) und zum Identifizieren eines Endoskopes (107) einer endoskopischen Vorrichtung (101) nach Anspruch 9, wobei das Computerprogrammprodukt Befehle aufweist, welche bei der Ausführung des Computerprogrammproduktes durch die Bild- und/oder Datenverarbeitungseinrichtung ein Erkennen der in der definierten Markierungszoomstellung erfassten optischen Markierung (121, 123, 125) in der Bildaufnahme, ein Zuordnen der erkannten, optischen Markierung zu einem bestimmten optischen Element und/oder zu einem bestimmten Endoskop und somit ein Identifizieren des bestimmten optischen Elementes und/oder des bestimmten Endoskopes und optional ein Ausgeben einer Benachrichtigung an den Benutzer der endoskopischen Vorrichtung (101) ausführt.

## Claims

1. Endoscopic device (101), in particular a medical or industrial endoscopic device, wherein the endoscopic device (101) has as components a light source, an optical fibre (119), a camera (103) and an endoscope (107) having an optical element (109, 113, 115) or a plurality of optical elements, wherein at least one optical element (109, 113, 115) has an optical marking (121, 123, 125) for verifying an identity of the optical element (109, 113, 115) and/or of the endoscope (107) such that the optical marking (121, 123, 125) can be detected by the camera (103) for verifying the identity and/or a configuration of the endoscopic device (101), **characterised in that** the camera (103) has a zoom lens (105) having an adjustment mechanism for changing a focal length such that the optical marking(s) (121, 123, 125) can be detected with the zoom lens (105) in a range from a proximal end to a distal end of the endoscope (107), each in a defined marking zoom position.

2. Endoscopic device (101) according to claim 1, **characterised in that** two optical elements, three optical elements (109, 113, 115) and/or more optical elements each have an optical marking.

3. Endoscopic device (101) according to claim 1 or 2, **characterised in that** the optical element and/or optical elements is or are an eyepiece (109), a lens, in particular a rod lens (113), and/or a cover glass (115).

4. Endoscopic device (101) according to any one of the preceding claims, **characterised in that** the zoom lens (105) can be can be adjusted, using the adjustment mechanism, such that, in an object zoom position for viewing an object (130) to be examined, an image from the camera (103) in the object zoom position is free of the optical marking(s) (121, 123, 125).

5. Endoscopic device (101) according to any one of the preceding claims, **characterised in that** the optical marking and/or optical markings is or are a line marking (123), a colour marking, a QR code (125), a serial number (121) and/or plain text.

6. Endoscopic device (101) according to any one of the preceding claims, **characterised in that** the optical marking (121, 123, 125) is etched, engraved, lasered, printed and/or written in and/or on the optical element(s) (109, 113, 115).

7. Endoscopic device (101) according to any one of the preceding claims, **characterised in that** the optical marking (121, 123, 125) is arranged in the centre, on the outer edge and/or transversely over a surface of the optical element(s) (109, 113, 115).

8. Endoscopic device (101) according to any one of the preceding claims, **characterised in that** the optical marking (121, 123, 125) has a dye, in particular a UV and/or fluorescence dye.

9. Endoscopic device (101) according to any one of the preceding claims, **characterised in that** the endoscopic device (101) is assigned an image and/or data processing system for evaluating and processing a recorded image and/or identifying the relevant optical marking (121, 123, 125) of the optical element and/or the optical elements (109, 113, 115), or the endoscopic device (101) has the image and/or data processing system.

10. Method (201) for verifying an identity of a component of an endoscopic device (101) according to any one of claims 1 to 9, having the following steps:
- configuring (203) the endoscopic device (101) by arranging and/or connecting the light source, the optical fibre (119), the camera (103) and the endoscope (107) having an optical element or a plurality of optical elements (109, 113, 115),
- focusing (205) the camera (103) on at least one optical marking (121, 123, 125) of one of the optical elements (109, 113, 115), wherein the zoom lens (105) detects the optical marking(s) (121, 123, 125) in a range from a proximal end to a distal end of the endoscope (107), each in a defined marking zoom position,
- recording (207) an image of the at least one optical marking (121, 123, 125) using the camera (103),
- analysing (209) the recorded image and identifying (211) the at least one optical marking (121, 123, 125) and/or the optical element (109, 113, 115) of the endoscope (107),
- repeating (213) the focusing (205), recording (207), analysing (209) and identifying (211) of a further optical marking (121, 123, 125) of a further optical element (109, 113, 115) and/or outputting (215) a result of the identifying (211) for a medical or industrial application of the configured endoscopic device (101).

11. Method (201) according to claim 10, **characterised in that** when focusing (205) the camera (103) on an object (130) to be examined, the optical marking(s) (121, 123, 125) is/are not visible.

12. Method (201) according to any one of claims 10 or 11, **characterised in that** the focusing (205) of the camera (103) is carried out automatically upon connection with the endoscope (107) or upon activation of the endoscope (107).

13. Computer program product for evaluating an image recording of an optical marking (121, 123, 125) of an optical element (109, 113, 115) and for identifying an endoscope (107) of an endoscopic device (101) according to claim 9, wherein the computer program product has instructions that, when the computer program product is executed by the image and/or data processing device, recognise the optical marking (121, 123, 125) detected in the defined marking zoom position in the image recording, assign the recognised optical marking to a specific optical element and/or to a specific endoscope and thus identify the specific optical element and/or the specific endoscope and optionally output a notification to the user of the endoscopic device (101).

## Revendications

1. Dispositif endoscopique (101), notamment dispositif endoscopique médical ou industriel, le dispositif endoscopique (101) présentant en tant que composants une source de lumière, un guide de lumière (119), une caméra (103) et un endoscope (107) avec un élément optique (109, 113, 115) ou plusieurs éléments optiques, au moins un élément optique (109, 113, 115) présentant un marquage optique (121, 123, 125) pour vérifier une identité de l'élément optique (109, 113, 115) et/ou de l'endoscope (107) de telle sorte que le marquage optique (121, 123, 125) peut être détecté par la caméra (103) pour vérifier l'identité et/ou une configuration du dispositif endoscopique (101), **caractérisé en ce que** la caméra (103) présente un objectif de zoom (105) avec un moyen de réglage pour modifier une distance focale de telle sorte qu'avec l'objectif de zoom (105), le marquage optique ou les marquages optiques (121, 123, 125) peuvent être détectés dans une zone allant d'une extrémité proximale à une extrémité distale de l'endoscope (107) respectivement dans une position de zoom de marquage définie.

2. Dispositif endoscopique (101) selon la revendication 1, **caractérisé en ce que** deux éléments optiques, trois éléments optiques (109, 113, 115) et/ou plusieurs éléments optiques présentent chacun un marquage optique.

3. Dispositif endoscopique (101) selon la revendication 1 ou 2, **caractérisé en ce que** l'élément optique et/ou les éléments optiques sont un oculaire (109), une lentille, notamment une lentille à tige (113), et/ou un verre de couverture (115).

4. Dispositif endoscopique (101) selon l'une quelconque des revendications précédentes, **caractérisé en ce que** l'objectif de zoom (105) peut être réglé au moyen du moyen de réglage de telle sorte que, dans une position de zoom d'objet pour observer un objet (130) à examiner, une image de la caméra (103) dans la position de zoom d'objet est exempte du marquage optique ou des marquages optiques (121, 123, 125).

5. Dispositif endoscopique (101) selon l'une quelconque des revendications précédentes, **caractérisé en ce que** le marquage optique ou les marquages optiques est ou sont un marquage à barres (123), un marquage de couleur, un code QR (125), un numéro de série (121) et/ou un texte clair.

6. Dispositif endoscopique (101) selon l'une quelconque des revendications précédentes, **caractérisé en ce que** le marquage optique (121, 123, 125) est décapé, gravé, gravé au laser, imprimé et/ou écrit dans et/ou sur l'élément optique ou les éléments optiques (109, 113, 115).

7. Dispositif endoscopique (101) selon l'une quelconque des revendications précédentes, **caractérisé en ce que** le marquage optique (121, 123, 125) est agencé au centre, sur le bord extérieur et/ou en travers d'une surface de l'élément optique ou des éléments optiques (109, 113, 115).

8. Dispositif endoscopique (101) selon l'une quelconque des revendications précédentes, **caractérisé en ce que** le marquage optique (121, 123, 125) présente un colorant, notamment un colorant UV et/ou fluorescent.

9. Dispositif endoscopique (101) selon l'une quelconque des revendications précédentes, **caractérisé en ce qu'**un dispositif de traitement d'images et/ou de données est associé au dispositif endoscopique (101) pour évaluer et traiter une image enregistrée et/ou identifier le marquage optique respectif (121, 123, 125) de l'élément optique et/ou des éléments optiques (109, 113, 115) ou le dispositif endoscopique (101) présente le dispositif de traitement d'images et/ou de données.

10. Procédé (201) de vérification de l'identité d'un composant d'un dispositif endoscopique (101) selon l'une quelconque des revendications 1 à 9, avec les étapes suivantes :
- la configuration (203) du dispositif endoscopique (101) en agençant et/ou en reliant la source de lumière, le guide de lumière (119), la caméra (103) et l'endoscope (107) présentant un élément optique ou plusieurs éléments optiques (109, 113, 115),
- la focalisation (205) de la caméra (103) sur au moins un marquage optique (121, 123, 125) de l'un des éléments optiques (109, 113, 115), l'objectif de zoom (105) détectant le marquage optique ou les marquages optiques (121, 123, 125) dans une zone allant d'une extrémité proximale à une extrémité distale de l'endoscope (107), respectivement dans une position de zoom de marquage définie,
- l'enregistrement (207) d'une image de l'au moins un marquage optique (121, 123, 125) au moyen de la caméra (103),
- l'analyse (209) de l'image enregistrée et l'identification (211) de l'au moins un marquage optique (121, 123, 125) et/ou de l'élément optique (109, 113, 115) de l'endoscope (107),
- la répétition (213) de la focalisation (205), de l'enregistrement (207), de l'analyse (209) et de l'identification (211) d'un autre marquage optique (121, 123, 125) d'un autre élément optique (109, 113, 115) et/ou l'émission (215) d'un résultat de l'identification (211) pour une application médicale ou industrielle du dispositif endoscopique configuré (101).

11. Procédé (201) selon la revendication 10, **caractérisé en ce que**, lors de la focalisation (205) de la caméra (103) sur un objet (130) à examiner, le marquage optique ou les marquages optiques (121, 123, 125) ne sont pas visibles.

12. Procédé (201) selon l'une quelconque des revendications 10 ou 11, **caractérisé en ce que** la focalisation (205) de la caméra (103) est effectuée automatiquement lors d'une liaison à l'endoscope (107) ou lors de la mise sous tension de l'endoscope (107).

13. Produit programme d'ordinateur pour évaluer un enregistrement d'image d'un marquage optique (121, 123, 125) d'un élément optique (109, 113, 115) et pour identifier un endoscope (107) d'un dispositif endoscopique (101) selon la revendication 9, le produit programme d'ordinateur présentant des instructions qui, lors de l'exécution du produit programme d'ordinateur par le dispositif de traitement d'images et/ou de données, permettent de reconnaître le marquage optique (121, 123, 125) détecté dans la position de zoom de marquage définie, 123, 125) dans l'enregistrement d'image, d'associer le marquage optique reconnu à un élément optique déterminé et/ou à un endoscope déterminé et ainsi d'identifier l'élément optique déterminé et/ou l'endoscope déterminé et, en option, d'émettre une notification à l'utilisateur du dispositif endoscopique (101).
